# EUROPEAN PATENT SPECIFICATION

(11) **EP 3 569 279 B1**
(45) Date of publication and mention of the grant of the patent: **30.12.2020**
(21) Application number: 19181644.6
(22) Date of filing: 08.03.2013
(51) Int. Cl.: A61M 25/00, A61M 25/10

(54) **BALLOON CATHETER WITH FLOATING HUB**
BALLONKATHETER MIT SCHWEBENDER NABE
CATHÉTER À BALLONNET À MOYEU FLOTTANT

(30) Priority: 09.03.2012 US 201261608927 P; 09.03.2012 NL 2008453
(43) Date of publication of application: 20.11.2019
(62) Document number(s) of the earlier application(s) in accordance with Art. 76 EPC: 13721138.9 / 2 822 629
(73) Proprietor: Clearstream Technologies Limited, Enniscorthy, County Wexford (IE)
(72) Inventor: Byrne, Pat, Enniscorthy, County Wexford (IE); Jensen, Angela Kay, Tempe, AZ 85281 (US); Schaffer, Andrew, Tempe, AZ 85281 (US)
(74) Representative: Hoffmann Eitle

(56) References cited:
- EP-A1- 0 850 659
- WO-A2-2006/135581
- WO-A2-2010/003135
- US-A1- 2009 171 278

## Description

### Technical Field

This disclosure relates generally to catheters for performing medical procedures, such as balloon angioplasty and, more particularly, to a catheter including a floating hub, which may be used for ensuring the proper alignment between a radiopaque identifier and a portion of the balloon, such as the working surface.

### Background of the Invention

Balloons are routinely used to resolve or address flow restrictions or perhaps even complete blockages in tubular areas of the body, such as arteries or veins. In many clinical situations, the restrictions are caused by hard solids, such as calcified plaque, and require the use of high pressures to compact such blockages. Commercially available balloons employ complex technology to achieve high pressure requirements without sacrificing the profile of the balloon. Besides high pressure requirements, the balloons should also be resistant to puncture, easy to track and push, and present a low profile, especially when used for angioplasty.

In clinical practice, angioplasty balloons are expanded from a deflated, folded state to an expanded state within a vessel to treat a target area, such as a portion of the circumferential inner wall I of a blood vessel V, as shown in Figures 1 and 2. The inflation of a balloon 12 with wall 28 is traditionally completed using an X-ray contrast agent CM along dimension DX to provide better visibility under X-ray or other form of radiography R during the interventional procedure, as illustrated in Figures 3 and 3a (which shows the intensity measured by a fluoroscope detector plate, FDP). Typically, a 70/30 percent mixture of contrast agent and saline is used to inflate the balloon during an angioplasty procedure.

In general, a desirable goal is to reduce inflation and deflation times required for balloons without sacrificing the profile of the balloons, especially for large volume balloons (which can require up to two minutes of inflation/deflation times with the contrast agent). Because of its relatively high viscosity, it would also be desirable to eliminate, or at least reduce the amount of, the contrast agent used in inflation/deflation of the balloons. The use of contrast agent prolongs the inflation/deflation times and also poses the risk of iodine exposure to patients sensitive to iodine. In this regard, a non-radiopaque substance could be used in lieu of the contrast agent, such as for example saline or carbon dioxide, but such substances are invisible during X- ray imaging, and thus do not enhance visibility.

Furthermore, the physician performing the angioplasty procedure should be able to locate the position of the uninflated balloon with accuracy, so that the balloon will be properly positioned once inflated. This is conventionally accomplished by attaching marker bands on the catheter shaft in the region corresponding to the balloon working surface. This "working surface" is the surface along the portion of the balloon that is used to achieve the desired treatment effect, such as contacting the calcified plaque (which surface in the case of a balloon having conical or tapering sections at the proximal and distal ends is typically co-extensive with a generally cylindrical barrel section).

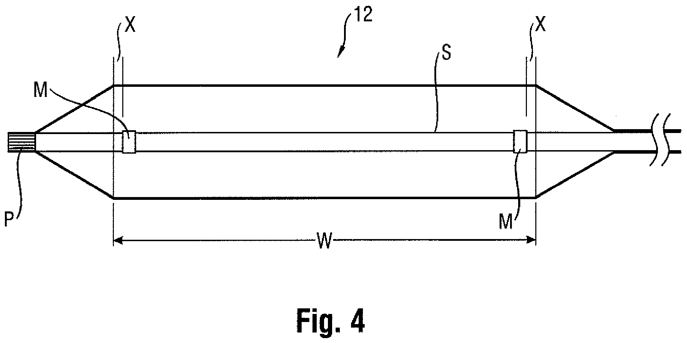

Misalignment of the marker bands during placement along the shaft sometimes results in their failure to correspond precisely to the extent of the working surface, as is shown in Figure 4 (note misalignment amount X between each interior marker band M carried by shaft S and working surface W of balloon 12, which also typically includes a radiopaque tip P at the distal end). Even upon exercising great care to position the markers properly on the underlying shaft in alignment with anticipated boundaries of the working surface when the balloon is inflated, there remains a tendency for mismatch due to several possible factors. One such factor may be the tolerance stack-ups arising as a consequence of the affixation of the balloon to the distal end of the catheter shaft. The balloon also has a tendency to grow in the longitudinal direction when inflated, especially with large and particularly long balloons. Another factor is the tendency of the portion of the catheter shaft within the balloon to bend or flex during inflation. This may lead to misalignment between radiopaque markers fixed to the shaft and the working surface.

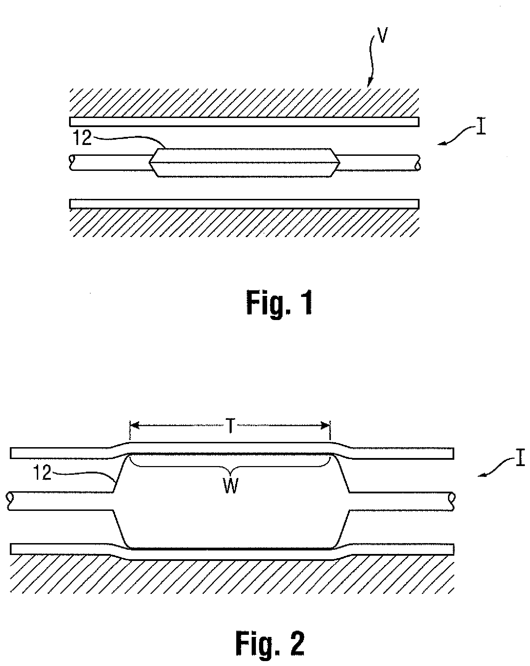

Whatever the cause, the resulting misalignment may prevent the clinician from accurately identifying the location of the working surface of the balloon during an interventional procedure. This may lead to a geographic misplacement, or "miss," of the intended contact between the target area T and the working surface W of the balloon 12 (see Figure 2). It is especially desirable to avoid such an outcome when the balloon is designed to deliver a payload (such as a drug, stent, or both) or a working element to a specified location within the vasculature, since a miss may prolong the procedure (such as, for example, by requiring redeployment of the balloon 12 or the use of another balloon catheter in the case of a drug coated balloon).

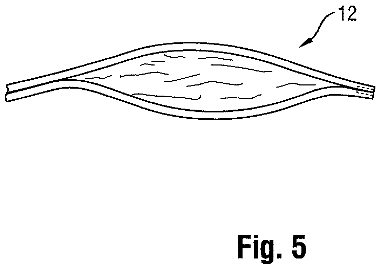

Upon deflation, the balloon may also be subject to a phenomenon known as "pancaking." In this condition, the balloon 12 folds down upon itself to a flattened state, as shown in Figure 5. This situation may cause the balloon to be viewed through fluoroscopy as perhaps still being in the inflated condition, since the full width of the balloon may still be perceived. This can give the clinician the false perception that the balloon remains inflated, when in fact it is not.

Accordingly, the need is identified for a balloon for which the working surface may be identified during an interventional procedure with enhanced precision. The solution would take into account the possible mismatch between fixed locations on the catheter shaft and the balloon to define the working surface. The improved identification may also allow for the better detection of the false perception of deflation caused by pancaking. Overall, procedural efficiency would be enhanced without remarkably increasing cost or complexity, and in a manner that can be applied to many existing catheter technologies without extensive modification.

WO 2010/003135 A2 discloses an apparatus that is operable in different modes to perform various functions for treating a body lumen.

### Summary of the Invention

An object of the disclosure is to provide a hub for a balloon for which the working surface may be identified during an interventional procedure with enhanced precision, as further disclosed in claim 1.

### Brief Description of the Drawings

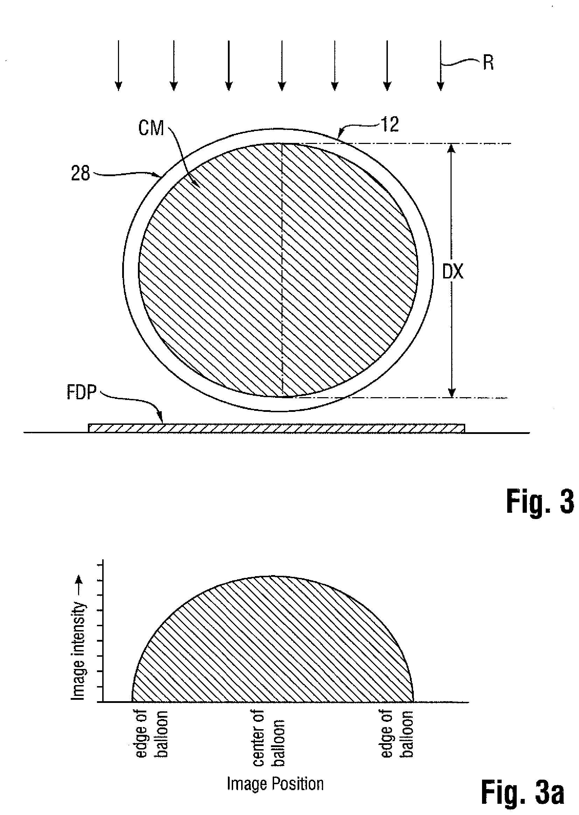

Figures 1-9 are illustrative of the background of the disclosure;

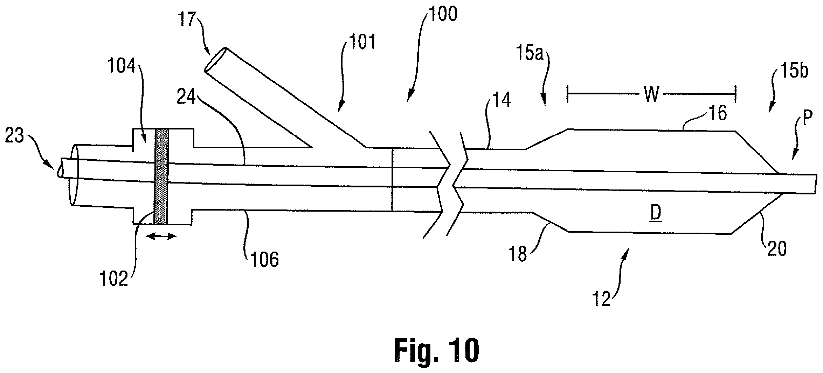

Figure 10 illustrates a first embodiment according to the disclosure;

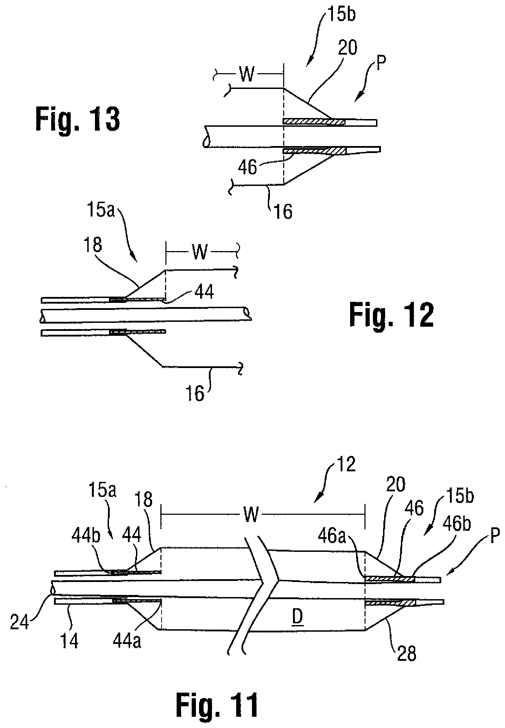

Figure 11 illustrates a second embodiment according to the disclosure;
Figures 12 and 13 are partial views of the Figure 11 embodiment;

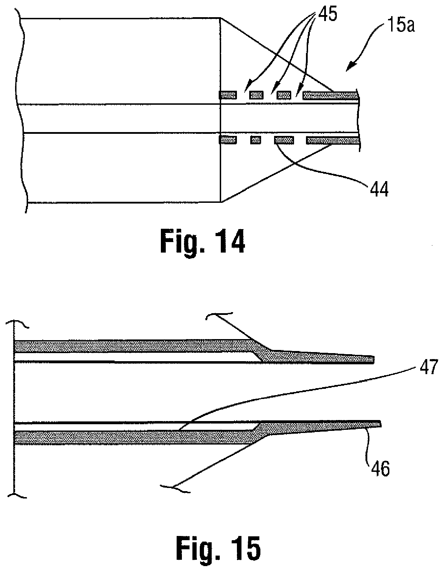

Figures 14 and 15 illustrate additional aspects of the disclosure;
Figure 15 illustrates the embodiment of Figure 14 in a folded condition; and

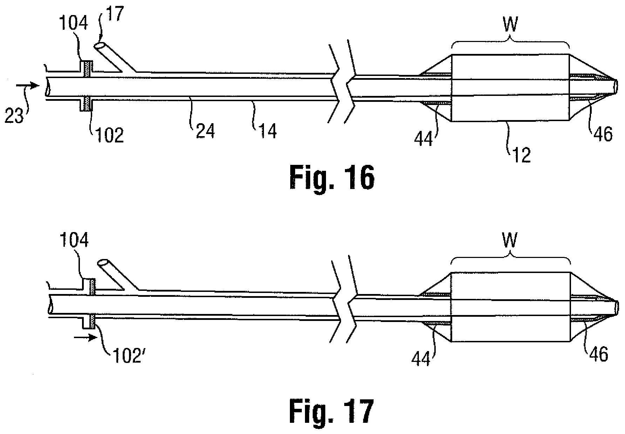

Figures 16 and 17 illustrate a further embodiment according to the disclosure.

### Modes for Carrying Out the Invention

The description provided below and in regard to the figures applies to all embodiments unless noted otherwise, and features common to each embodiment are similarly shown and numbered.

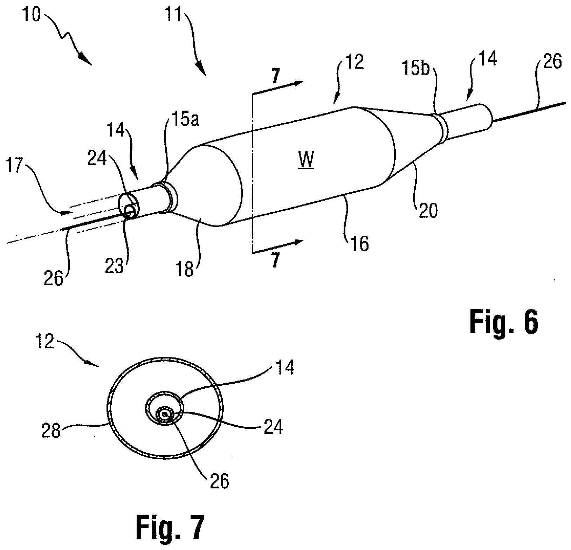

Provided is a catheter 10 having a distal portion 11 with a balloon 12 mounted on a catheter tube 14. Referring to Figures 6, 7, and 8, the balloon 12 has an intermediate section 16, or "barrel," and end sections 18, 20. In one embodiment, the end sections 18, 20 reduce in diameter to join the intermediate section 16 to the catheter tube 14 (and thus sections 18, 20 are generally termed cones or cone sections). The balloon 12 is sealed at balloon ends (proximal end 15a and distal end 15b) on the cone sections 18,20 to allow the inflation of the balloon 12 via one or more inflation lumens 17 extending within catheter tube 14 and communicating with the interior of the balloon 12.

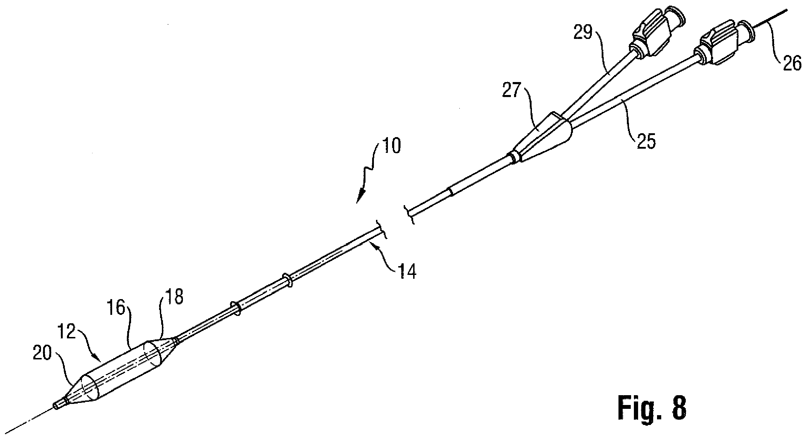

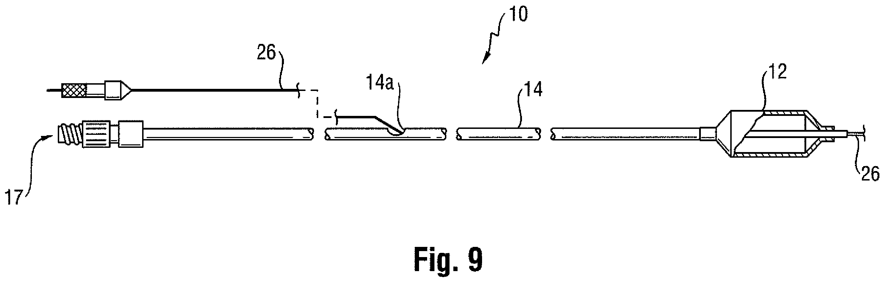

The catheter tube 14 also includes an elongated, tubular shaft 24 forming a guidewire lumen 23 that directs the guidewire 26 through the catheter 10, and along the distal end of which the balloon 12 may be located. As illustrated in Figure 8, this guidewire 26 may extend through the proximal end of the catheter 10 and a first port 25 of a connector 27 into the lumen 23 to achieve an "over the wire" (OTW) arrangement, but could also be provided in a "rapid exchange" (RX) configuration, in which the guidewire 26 exits a lateral opening 14a closer to the distal end (see Figure 9) or else is fed through the tip distally of the balloon 12 (not shown). A second port 29 may also be associated with catheter 10, such as by way of connector 27, for introducing a fluid (e.g., saline, a contrast agent, or both) into the interior compartment of the balloon 12 via the inflation lumen 17.

Balloon 12 may include a single or multi-layered balloon wall 28 forming the interior for receiving the inflation fluid. The balloon 12 may be a non-compliant balloon having a balloon wall 28 that maintains its size and shape in one or more directions when the balloon is inflated. Examples of non-compliant balloons may be found in U.S. Pat. No. 6,746,425 and Publication Nos. US 2006/0085022, US 2006/0085023 and US 2006/0085024. The balloon 12 in such case also has a pre-determined surface area that remains constant during and after inflation, also has a pre-determined length and pre-determined diameter that each, or together, remain constant during and after inflation. However, the balloon 12 could be semi-compliant or compliant instead, depending on the particular use.

One embodiment for achieving the desired features is by arranging the shaft 24 in a manner that allows it to move a predetermined amount under a restrained condition in order to align the working surface W with particular location, such as the position of one or more radiopaque identifiers. In one embodiment, as shown schematically in Figure 10, the catheter 100 includes a seal 102, such as may be provided by one or more O-rings or the like, at or near a proximal end of the inner shaft 24. The seal 102 is adapted for positioning in a recess 104 formed in a receiver 101 for receiving the shaft 24, such as hub 106, which is shown as being oversized for purposes of illustration. The recess 104 is oversized so as to allow movement of the seal 102 and thus shaft 24 relative to the tube 14 within inflation lumen 17, which is in turn connected to the proximal end of the balloon 12. Other than at the distal end 15b of balloon 12 and the seal 102, the shaft 24 is not connected to any other structure in the catheter 10, and thus can move to and from a distance in the longitudinal direction corresponding to the length of the recess 104.

Turning to Figures 11-15, the balloon 12 may include a radiopaque identifier separate from the shaft 24 to allow for identification of a particular location under fluoroscopy. In one embodiment, this identifier is provided in the form of an insert 44. This insert 44 may be tubular in form, and extend in a spaced apart manner along and generally coaxially with the shaft 24 extending through the interior compartment of the balloon 12, thus forming a sleeve. A free end portion 44a of the insert 44 is located at a position aligned with the edge or extent of the working surface W at the proximal end 15a, while the opposite, fixed end portion 44b is connected to the balloon 12, such as at the point where it attaches to the tube 14 at this proximal end. The connection between the insert 44 and the balloon 12, tube 14, or both, may be one or more of an interference fit, bonding (using an adhesive, welding, etc.), friction, or other like manners of forming a secure arrangement that does not permit relative movement of two distinct parts when connected.

The insert 44 may also be adapted so as to minimize interference with the inflation of the balloon 12. For example, as shown in Figure 14, the insert 44 may be provided with one or more perforations 45. These perforations 45 prevent the insert 44 from serving as a barrier that would retard the inflation fluid emanating from the tube 14 from readily flowing into the interior compartment of the balloon 12, and thus help to preserve the desired short inflation times. Indeed, the presence of such perforations 45 may allow the inserts 44 and 46 to be combined into a single unitary piece of material extending continuously between the proximal and distal ends 15a, 15b of the balloon 12.

A second, at least partially radiopaque insert 46 may also be provided at the distal end 15b including the conical section 20. In one embodiment, this insert 46 is arranged at the location where the distal end 15b of the balloon 12 is secured to the shaft 24 to form the tip P (which as noted may also be radiopaque). As shown in Figure 15, this insert 46 may be connected to the shaft 24, or may be embedded within the material forming the corresponding distal end 15b of the balloon 12 (especially if a multi-layered arrangement is used to form the balloon wall 28).

Other types of identifiers may also be used instead of or in addition to the particular inserts 44, 46 shown. Details may be found in commonly filed patent applications, entitled **"**MEDICAL BALLOON INCLUDING RADIOPAQUE INSERT FOR PRECISELY IDENTIFYING A WORKING SURFACE LOCATION**,"** for inventors Pat Byrne, Sean Wall, Angela Jensen, Andrew Schaffer, Angela Crall.

In any case, the proximal end portion 46a of the insert 46 terminates at the distal edge of the working surface W, so as to assist in the identification process. The proximal end portion 46a of this insert 46 extending within the interior compartment of the balloon 12 may be spaced from the shaft 24 (note gap 47 in Figure 15) so as to be less susceptible to any misalignment caused by bending. This end 46a may be partially or entirely radiopaque. This radiopaque end portion 46a provides an indication of the distance between the distal edge of the working surface W and the distal end 15b of the balloon 12. Also, the distal end portion 46b of the insert 46 may be formed with the tip P as a unitary, continuously radiopaque structure (for example, with different radiopaque qualities (patterns, shapes, densities, etc.)), if desired to allow for ready identification of these components.

With reference now to Figures 16 and 17, it can be understood that the seal 102 provides the desired closed lumen 17 to receive the inflation fluid. On inflation of the balloon 12, such as in a vessel V, any tendency of the shaft 24 to bend and cause misalignment has no impact on the location of the radiopaque identifier, such as inserts 44, 46. This is because the insert 44 at the proximal end 15a is separate from the shaft 24, and the insert 46 at the distal end 15b of the balloon 12 may move along with the shaft 24, which is free to move in the longitudinal direction until the seal 102 reaches the distal end of the recess 104 (note position 102' in Figure 17). This tends to eliminate any tendency of the shaft 24 within the balloon 12 to bend, and even with minor bending, the inserts 44, 46 are generally separate from the intermediate portion of the shaft 24 most susceptible to bending. The result is a more precise alignment of the radiopaque identifier with the target treatment area and ultimately with the working surface W of the balloon 12, and without regard to any bending of the shaft 24.

Balloons 12 that carry one or more surface elements, such as a payload (drug, stent, or both) or a working implement (cutter, focused force wire, or the like) into the vasculature may also benefit from the foregoing description of marking techniques. For example, as shown in Figure 10, a balloon 12 including a defined working surface W may include a portion coated with such a drug D, such as one designed for achieving a desired therapeutic effect when applied to the interior of the vessel. The drug D may be applied to the inflated balloon as part of the manufacturing process, and prior to folding for insertion in the vasculature. The clinician may thus with the benefit of a fluoroscope determine the precise positioning of the working surface W prior to inflating the balloon 12 in the vasculature to deliver the drug D to the desired location and provide the desired treatment regimen.

## Claims

1. A hub (106) for a balloon catheter (10) having an elongated, tubular shaft (24) extending in a longitudinal direction, said shaft (24) having a proximal end and a distal end, and an inflatable balloon (12) connected to the distal end of the shaft for being inflated by an inflation fluid, comprising:
a body including a receiver (101) for receiving a proximal portion of the shaft (24) and adapted for allowing the shaft (24) to move relative to the receiver in at least the longitudinal direction; and
a stop (102) for restraining the movement of the shaft relative to the body in the longitudinal direction;
the receiver comprising a recess (104) for receiving the stop, wherein the stop forms a seal (102) with the recess (104) of the receiver for receiving the stop to prevent the inflation fluid from passing,
**characterised in that** said recess (104) has a dimension in the longitudinal direction that is greater than a corresponding dimension of the stop (102).

2. The hub of claim 1, wherein the body includes a guidewire port (25) arranged in communication with the receiver, and further including an inflation port (29) for introducing the inflation fluid for inflating the balloon.

3. The hub of claim 1 or 2, wherein the stop comprises an O-ring.

## Patentansprüche

1. Nabe (106) für einen Ballonkatheter (10), aufweisend einen länglichen, röhrenförmigen Schaft (24), der sich in eine Längsrichtung erstreckt, wobei der Schaft (24) ein proximales Ende und ein distales Ende aufweist, und einen aufblasbaren Ballon (12), der mit dem distalen Ende des Schafts verbunden ist, um mit einem Aufblasfluid aufgeblasen zu werden, umfassend:
einen Körper, der eine Aufnahme (101) zum Aufnehmen eines proximalen Abschnitts des Schafts (24) einschließt und angepasst ist, um es dem Schaft (24) zu ermöglichen, sich in Bezug auf die Aufnahme in mindestens die Längsrichtung zu bewegen; und
einen Anschlag (102) zum Einschränken der Bewegung des Schafts in Bezug auf den Körper in die Längsrichtung;
wobei die Aufnahme eine Aussparung (104) zum Aufnehmen des Anschlags umfasst, wobei der Anschlag eine Dichtung (102) mit der Aussparung (104) der Aufnahme zum Aufnehmen des Anschlags bildet, um zu verhindern, dass das Aufblasfluid durchläuft,
**dadurch gekennzeichnet, dass** die Aussparung (104) in der Längsrichtung eine Abmessung aufweist, die größer ist als eine entsprechende Abmessung des Anschlags (102).

2. Nabe nach Anspruch 1, wobei der Körper einen Führungsdrahtanschluss (25) einschließt, der in Kommunikation mit der Aufnahme eingerichtet ist, und weiter einschließend einen Aufblasanschluss (29) zum Einführen des Aufblasfluids zum Aufblasen des Ballons.

3. Nabe nach Anspruch 1 oder 2, wobei der Anschlag einen O-Ring umfasst.

## Revendications

1. Raccord (106) pour un cathéter à ballonnet (10) présentant une tige tubulaire allongée (24) s'étendant dans une direction longitudinale, ladite tige (24) présentant une extrémité proximale et une extrémité distale, et un ballonnet gonflable (12) relié à l'extrémité distale de la tige pour être gonflé par un fluide de gonflage, comprenant :
un corps incluant un récepteur (101) pour recevoir une partie proximale de la tige (24) et adapté pour permettre à la tige (24) de se déplacer par rapport au récepteur dans au moins la direction longitudinale ; et
une butée (102) pour limiter le déplacement de la tige par rapport au corps dans la direction longitudinale ;
le récepteur comportant un évidement (104) destiné à recevoir la butée, dans lequel la butée forme un joint d'étanchéité (102) avec l'évidement (104) du récepteur destiné à recevoir la butée pour empêcher le fluide de gonflage de passer,
**caractérisé en ce que**
ledit évidement (104) présente une dimension dans la direction longitudinale qui est supérieure à une dimension correspondante de la butée (102).

2. Raccord selon la revendication 1, dans lequel le corps inclut un orifice de fil de guidage (25) agencé en communication avec le récepteur, et incluant en outre un orifice de gonflage (29) pour introduire le fluide de gonflage pour gonfler le ballonnet.

3. Raccord selon la revendication 1 ou 2, dans lequel la butée comprend un joint torique.
